Europäisches Patentamt

European Patent Office

Office européen des brevets

(19)

(11) **EP 1 345 137 A1**

(12) **EUROPEAN PATENT APPLICATION**
published in accordance with Art. 158(3) EPC

(43) Date of publication:
**17.09.2003 Bulletin 2003/38**

(21) Application number: **00978042.0**

(22) Date of filing: **30.11.2000**

(51) Int Cl.⁷: **G06F 17/60**

(86) International application number:
**PCT/JP00/08489**

(87) International publication number:
**WO 02/044967 (06.06.2002 Gazette 2002/23)**

(84) Designated Contracting States:
**AT BE CH CY DE DK ES FI FR GB GR IE IT LI LU MC NL PT SE**

(71) Applicant: **Hitachi, Ltd.**
**Chiyoda-ku, Tokyo 101-8010 (JP)**

(72) Inventors:
• **YASUDA, Kenji, c/o Instruments of Hitachi, Ltd.**
**Hitachinaka-shi, Ibaraki 312-0033 (JP)**

• **TAKEGAWA, Yuichi,**
**c/o Instruments of Hitachi, Ltd.**
**Hitachinaka-shi, Ibaraki 312-0033 (JP)**

(74) Representative: **Strehl Schübel-Hopf & Partner**
**Maximilianstrasse 54**
**80538 München (DE)**

(54) **METHOD AND SYSTEM FOR PROVIDING GENE ANALYSIS INFORMATION AND AUTHENTICATION IDENTIFICATION METHOD**

(57)    A service of analyzing a homo gene is offered in the form conforming to the requirement of the customer. A service of carrying out gene analysis required by the customer through an electronic request method. A gene info rmation access center is set up, where the gene and its analysis information of the customer is stored. The gene is analyzed in response to the request of the customer and the result is provided. Information meeting the needs of the customer from among a huge amount of information on genome genes is provided with time and in phase.

*FIG. 1*

**Description**

TECHNICAL FIELD

The present invention relates to method and system for offering gene analysis information, and attestation identification method of a gene donor by using these method and system.

BACKGROUND ART

The Japanese Patent Application Laid-Open No. 2000-60553 relates to an apparatus for processing the motif extract of the gene and a method of processing the motif extract of the gene, and a recording medium in which the processing program of the motif extract of the gene is recorded. The processing apparatus for the motif extract of the gene includes a data input part where at least one gene sequence information is input, a data edit part where various edits and assignment of the range of motif extract, etc. are done to the input gene sequence information, a motif extract part where the motif of gene is automatically extracted from the input gene sequence information or the specified range of motif extract, an extract result processing part where the display of extracted motif and the registration to a motif data base, etc. are done, and a motif retrieval part where the gene sequence information in a part of which the extracted motif is contained is retrieved from a gene sequence information data, wherein the apparatus further includes a mechanism for adding the gene sequence information retrieved by the motif retrieval part to gene sequence information input from the data input part by inputting the retrieved gene sequence information to the data edit part.

There are the Japanese Patent Application Laid-Open No. 09-245107 and the Japanese Patent Application Laid-Open No. 11-149453, etc. as a well-known example which uses the gene to identify the attestation.

DISCLOSURE OF INVENTION

The problems of the present invention is in providing the method of offering information and its achievement means in view of the characteristics of gene information, wherein the information corresponds to various requests of the customer, by designing the mechanism providing it to the customer to whom gene information was confirmed in various forms and with time, and by continuously managing the supply of gene information over the long term.

The customer and the organization of the gene management are connected by an electronic means for transmitting information such as the Internet etc. are made electronic in the present invention to achieve the above-mentioned object. Further, the following business forms are designed, 1) a means for securing an individual secret, 2) provision of the phased information according to needs of the customer (including the medical treatment organization, the same as follows), and 3) gene preservation for a long term, information provision having the function to provide the information at any time for the period. An organization is set, in which customer's gene is collected and analyzed, and the storage of gene, and the provision and management of the gene information are performed in the lump, when the gene information (including the information which the obtained gene information is processed in this case) is provided. As a method of offering gene information from this organization, three phases of a short term, a middle term, and a long-term are set from a viewpoint of the emergency of information, the time required to analyze, and the time required to interpret the result and acquire the related information. Information can be provided even by the behavior comparison result of the restriction enzyme cleavage fragment, etc. though the gene information to be offered is mainly based on the sequence information of the base on the gene chain which composes genome. The base sequence is analyzed by a DNA analysis device usually used. Various el ectrophoresis apparatuses, DNA sequencers, and DNA chip analysis devices are chiefly used. However, it should be not limited to these technologies. Gene information on a normal homo and polymorphic information are compared with the information on the gene base sequence, the characteristic of customer's gene is derived, the influence and the mutation degree are evaluated, and the answer is provided in the form demanded by the customer. Demands of the customer diverge according to the importance degree based on the time required to obtain information first. If the gene information relates to immediate disease and treatment, the customer wants to obtain the result immediately. Then, Requests getting in three phases are set according to the easiness of gene analysis information and the length of the analytical time required.

First, the range of one month is set as a short term, and the gene which can be analyzed within the range and the information are offered. When information will be needed in a short term, the information is offered on the assumption of the cases based on the needs on the customer side such as the pre -acquirement of the information before the treatment, the cancellation of uneasiness and misgivings about the gene, and the cancellation of misgivings about states of the pregnancy and genetic diseases, in addition to the correspondence to the gene analysis of which the time required is short. The genome DNA is steady, and the needs that an urgent information supply is required to diagnose is few. However, there is one to require the emergency in the customer's needs. Moreover, the period of about six months is set in a mid -term analysis. The gene analysis which does not require the emergency and the gene analysis which requires time for the analysis such as life-style-relevant disease related genes, chronic ailment related gene, drug metabolism related gene, and SNP (monobasic polymorphous) based on two or more gene factors are carried out.

In a long-term analysis, the information is not provided by analyzing an only specific disease and gene characteristic, but the gene information is provided to the customer by analyzing the base sequence and its characteristic over the entire gene of the customer. This course requires occasionally a long term of one year or more to obtain all analytical results. Therefore, the analytical results are provided one by one. If the elucidation of a new gene advances to as the gene research progresses, an analysis request of the customer who requires the new elucidation can deal in such a long-term analysis.

The gene analysis is executed if the charge is presented to the customer based on the phase divided by the period of time of such information supply, the variety of the information supply based on the phase, and the information analysis charge, and it is accepted.

The information on gene DNA basically includes sequence information on four kinds of bases. The function part of the gene is distinguished from a specified part of this sequence. Namely, an object of the gene information service is to clarify an individual gene characteristic. Because the gene information has large capacity, the use of the electronic information network spread widely like the Internet is the best to transmit the information to the customer. As a result, a prompt information transmission can be done, and the two-way communications becomes easy.

BRIEF DESCRIPTION OF DRAWINGS

Fig.1 is an information route chart in an embodiment.
Fig.2 is a view showing the transition of the customer comput er screen.
Fig.3 is a view showing an example of the customer identification access screen.
Fig.4 is a view showing an example of the offer service screen.
Fig.5 is a view showing an example of the screen of a short-term analytical menu.
Fig.6 is a view showing an example of the screen of a mid-term analytical menu.
Fig.7 is a view showing an example of the screen of a long-term analytical menu.
Fig.8 is a view showing an example of the screen of the information request menu.

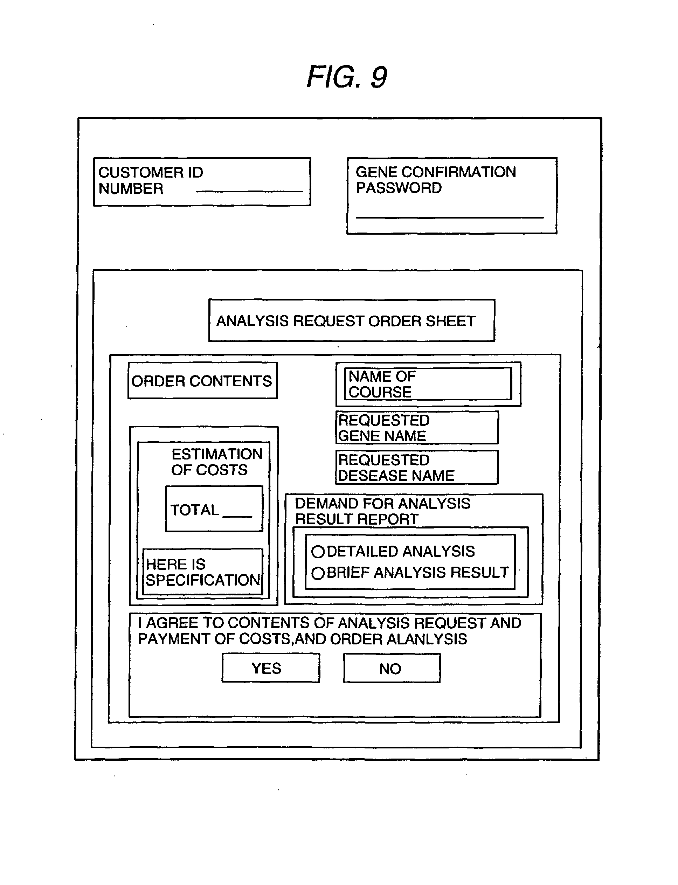

Fig.9 is a view showing an example of the analytical order screen from the customer.
Fig.10 is a view showing an example of the screen of an analytical result.
Fig.11 is a view showing an example of the screen of mediation and link information.
Fig.12 is a view showing an example of the screen of the explanation for the customer.
Fig.13 is a view showing the account payment method.

BEST MODE FOR IMPLEMENTING THE INVENTION

Hereafter, the present invention will be explained based on the embodiment.

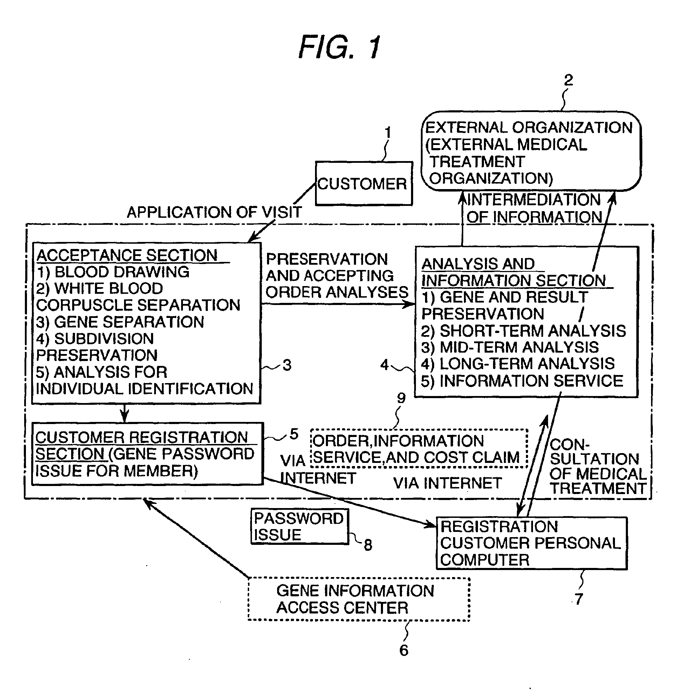

The embodiment according to the present invention is described in Fig.1 in schematic form. In the present invention, the mechanism which accomplishes a main function is gene information access center 6. All roles of the gene obtaining from the customer, analysis, data storage, data transmission, an analytical information service, and the report and mediation, etc. to the medical treatment organization are performed in this organization. The information transmission with the customer is done through the electronic computer network and the Internet.

Gene information access center 6 first requests the gene to for instance new customer 1 who wishes to be served. The usual gene separation is carried out by using the method described in the chapter of the Japanese Association of Medical Technologists "Clinical examination, Gene and Chromosome examination textbook" (issued by Kindai Publication in 1998), etc.

Acceptance section 3 in the gene information access center works as follows. Customer's peripheral blood is collected by an injector etc.; the buffer solution which dissolves the red blood corpuscle is added. The solution is centrifugally separated for five minutes at 3000 revolutions per minute by a centrifugal separator, and then the supernatant liquid is thrown away. The deposit is suspended in the buffer after the supernatant liquid becomes clean, proteolytic enzyme proteinaze K and surface active agent SDS are added, and digested. The saturation phenol liquid is added to this, the supernatant is separated, and DNA contained in this is precipitated with the ethanol. Afterwards, the supernatant liquid is centrifugally separated, the ethanol is evaporated finally, too and is dissolved in the tris-EDTA buffer solution. In addition, it is divided into a lot of preservation containers little by little, and they are preserved in the low temperature frozen warehouse of -80°C or less. The gene of each person is numbered, and kept individually. The refined gene is moved to analysis and information section 4, and kept at the ultra low temperature till the analytical work. Next, in acceptance section 3, the little preserved gene of 5-10μ gram is melted and analyzed to do the gene identification of the customer who requests for the analysis with a high degree of accuracy. DNA is cut into a suitable fragment by the restriction enzyme, and DNA fragment which contains the sequence for identification is amplified by adding the primer for individual identification. It is amplified by the conventional PCR method (Roche Co.) etc. as an amplification method, and the base sequence is read. It is requested to set the base sequence of the fragment with a large individual change as an identification code based on the sequence information in such a gene segment It is possible to make the identification code by combining base sequence information on two or more short fragments. The base sequence which includes 12 places or more, the parts where four-kind substitution of adenin (A), thymine (T), guanine (G), and cytosine (C) occurs easily is selected, and used for the identification of the individual. If the gene sequence part to which four kinds of base change at the probability of 25% is used, and the password which contains these change parts at 12 places or more is used for identification for instance, $4^{12}$=16,777,216 kinds of combinations are thought. Therefore, only the base sequence of the same combination with the possibility of about one person to every 16,000,000 people or more same can occur. Accordingly, the customer can be identified very selectively. If this probability is high still enough, it may become possible to obtain the possibility of about one person to every 100,000,000 people or more by increasing the number of base sequence parts of four-kind mutations to be chosen, or it may choose base sequence with lower probability. If the identification password is issued so as to include the high frequency mutation base parts at 15 places or more, the individual who has same identification code on the earth is gone, when judged from the population on the present earth. In the special case like the twin, it is possible to identify them by setting the code number having another one digit. Customer registration section 5 transmits the individual identification code number of the gene-origin decided like this to the customer as password 8, in order to confirm the access on the Internet customer screen. An example of such a password is shown in Table 1. In Example 1, the individual is identified by 40 alphabetic characters of A, T, G and C. The alphabet of X and Y at the end was given for the distinction though Examples 2 and 3 were identical twin's gene password. With regard to the length of the password like this, the decision method can be chosen, in which the passwords does not overlap based on the appropriate mutation rate and its base. The password may be divided suitably as shown at Example 4 or it may be used together with other signs, because it is not easy to memorize when the password is too long. Further, the combination of two or more passwords may be also used. However, the base sequence part adopted for the password is chosen so that there is no repetition.

Table 1

| Example Of Password | |
|---|---|
| Customer | Example of password |
| Example 1 | AATGCTAGCGGTAATCAATCGTGGCCTAACCGATGATCAG |
| Example 2 | GCGTATAGCGGTAAGCTCATGTCTGACTGGTACTGATCGTX |
| Example 3 | GCGTATAGCGGTAAGCTCATGTCTGACTGGTACTGATCGTY |
| Example 4 | AATGC-TAGCG-GTAAT-CAATC-GTGGC-CTAAC-CGATG-ATCAG |

A method of offering gene analysis information is achieved according to the communication between customer 1 and an organization. The organization is set up to execute the following processes; transmitting the information based on customer's specific gene information; collecting customer's gene, separating it, keeping it, and analyzing it properly when it is necessary; selecting in phase and with time and accepting an order based on the selection by the customer; transmitting the analytical result to the customer; keeping the customer's gene information for a long term; and exchanging the information with external organization 2 (for instance, external medical treatment organization) according to the demand from the customer.

Gene information is composed of the base sequence of DNA. The kind, the number, and gene associated diseases information on an analysis gene are contained for the period which the range of the selection to the information requires to obtain information.

The gene information is composed of the base sequence of DNA.

The range of selection of the information service is set so as to include the period of time required to acquire the information, the kind and number of the gene to be analyzed, and the gene related disease information.

A computer (personal computer) at customer registration section 5, a computer (personal computer 7) for the registered customer (gene donor), and a computer (personal computer) at analysis and information section 4 are connected by way of the Internet. Customer 1 can consult external organization 2 about the medical treatment by using registered customer's personal computer 7.

Thus, transmission of information, selection of the analytical content, order, and account payment (order, information service, and cost claim) 9 are performed through the electronic network.

The organization issues the password which combines two or more gene base sequence signs to the customer in order to perform the attestation identification by customer's gene.

Gene information access center 6 is the organization which operates as a core of the present invention as mentioned above, and manages the send and the receive of gene to and from the customer, the storage and the analysis of the gene, the acceptance of order and the result report of the gene analysis, the send and the receive of accounts, and customers' gene information. The exchange of the information and the communications between customer 1 and gene information access center 6 can be performed promptly and safely by using the network spread widely such as the Internet etc. It is required to manage strictly the information, because of the organization that manages the gene information that is extremely important individual information. It becomes possible to access the obtained gene information only when the customer agrees or only when the manager requested gives permission. The password based on customer's specific gene information is issued by the gene information access center, and the analysis is carried out after the password confirmation is completed (preservation and accepting order analysis). If the contact with the splitting ferment is refused, the gene DNA provided by the customer can be preserved stably for a long term, and can answer the request for the gene information from the customer for years. It is also possible to obtain the whole image of individual gene information by combining the gene information obtained gradually.

The disease that is the analysis object is following. Phenylketonuria, sickle -cell anemia, alpha-1-antitrypsin deficiency, citrullinemia, hemophilia, von Willebrand disease, antithrombin III anomalous syndrome, urea cycle enzyme deficiency, arginosuccinic acid urine syndrome, glycogenosis, mucopolysaccharidosis, Gaucher's disease, infantile amaurotic familial idiocy, Sandhoff disease, Lesch -Nyhan syndrome, APRT deficiency, adenosine deaminase anomalous syndrome, xeroderma pigmentosum, hypercholesterolemia, Tangier disease, Du Channe type myotrophia dystonica, a mitochondrion brain muscle syndrome, Kearns-Sayre syndrome, MELAS syndrome, MERRF syndrome, amyloid neuropathy, Alzheimer's disease, HCHWA-D, retinitis pigmentosa, pus bulla fibrosing disease, chronicity granuloma, polyposis coli, neurofibromatosis, Wilms tumor, and Li-Fraumeni syndrome, etc. These are only the one understood now, and the disease that derives from the gene displacement further increases as the research progresses. If immediate diagnosis is necessary when the genetic disease cause gene is analyzed, it is important to have a set of serious genetic disease cause gene analyzed first.

Moreover, the cause is complex and compositive in the life custom diseases (diseases such as diabetes mellitus, hypertension, and hyperlipemia which are caused by adult life customs) and chronic diseases (hepatitis, nephritis, and immunological disease, etc.). Most diseases are still at the research phase (referring to "Gene diagnosis introduction" written by Hiroshi Nojima, Youdo Co. 1992). However, it seems that the elucidation of the cause gene and the generation mechanism will be elucidated by the progress of a gene research of the disease in the future. Therefore, the gene information access center can offer the disease related gene information about these complicated diseases. The relationship between disease and monobase polymorphous SNP (Single Nucleotide Polymorphism)of the ge ne which seems to relate to the diathesis and the effect of the drug adverse reaction is hardly understood. If it is possible to provide as gene information, it becomes effective to treat the customer 's disease. Because the gene information controls the his/her biological function during all his/her life, it is also important to provide necessary gene information at each phase of his/her life. The long-term analysis can answer the demand of the customer who wants to clarify an individual inherited characteristic which does not relate directly to the disease in request. The gene information access center adds attached information to the gene analysis result based on various factors such as customer's job, the age, sex, contracted clinical histories, and the living conditions for a preventive object of the disease. The short -term, the mid-term, and the long-term are relative divisions. These fixed periods change according to the gene.

Therefore, an analytical menu can be frequently updated, if necessary.

The password for the customer identification is indispensable for secret maintenance. Using the password decided from customer's gene in the present invention enabled the person in question to recognize absolutely though it was also possible to specify the arbitrary password. The password is a code for the individual identification composed of four kinds of alphabets of A, T, G, and C which is basically the abbreviation of the base, and a lot of numbers of digits rises the identification degree. However, identification becomes difficult for multi child siblings more than a monovular twin or the persons whose genes are extremely similar. For this case, the alphabet is added further to distinguish. It is preferable to decide which gene sequence part to be assumed the sequence of the password according to the magnitude of the propability of mutation. The password is made by choosing a plurality of base sequences including the sequences of the circumference of the sequence where the change of the base is extremely high in each individual. If the part with high degree of variability is used, a different password can be decided to everyone by selecting the same gene sequence part to a lot of people. If the password like this into which individual gene information is taken is used, the decipherment becomes difficult and secret maintenance becomes easy. It should be confirmed that the password is different from the existing gene password before issued.

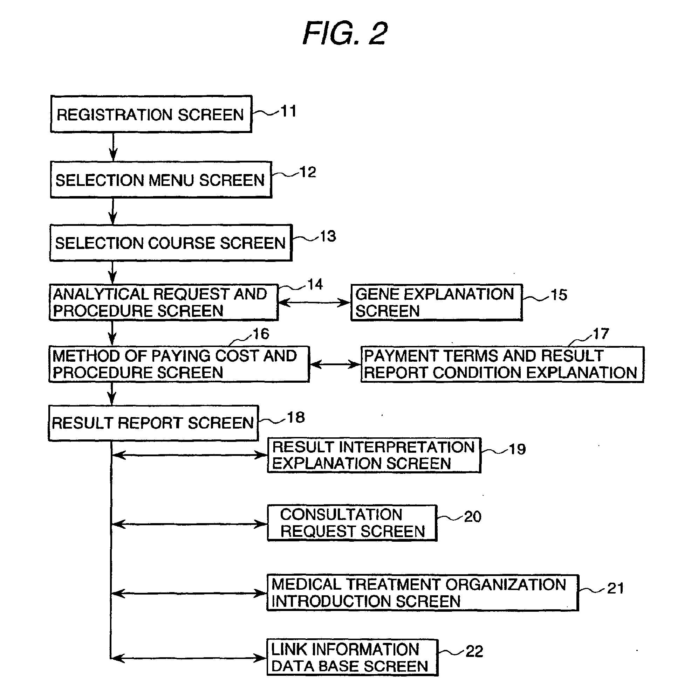

Fig.2 is a view showing the transition of the customer computer screen of registration customer personal computer 7. In this figure, selection menu screen 12 is made to appear from registration screen 11 to display selection course screen 13. Analysis requests and orders are performed from analysis request and order screen 14 with reference to gene explanation screen 15. Payment terms and result report condition explanation 17 are received, and various cost payment procedures are done from the method of paying cost and the procedure screen 16. Result report screen 18 is di splayed, and the result interpretation explanation, the consultation, the medical treatment organization introduction or the storage and keeping into the link information data base are performed with reference to result interpretation explanatory screen 19 , consultation request screen 20, medical treatment organization introduction screen 21 or link information data base screen 22.

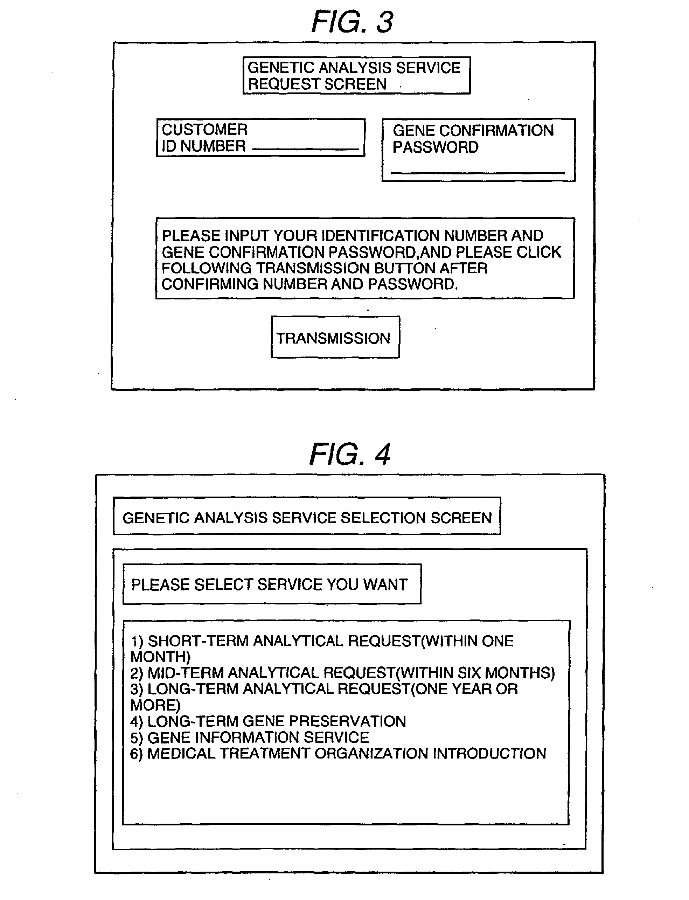

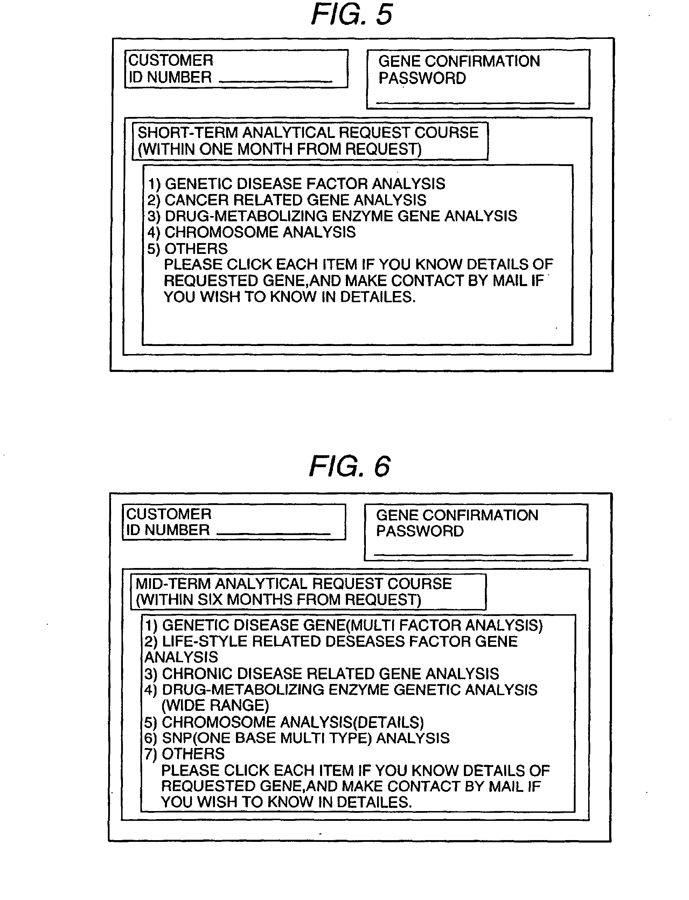

The customer confirmation collation work shown in Fig.3 is started first when the message of the service request from the customer is transmitted to customer registration section 5 at the gene access center, and a menu screen of the gene analysis service appears next when the confirmation is completed. Next, the display of the selection course shown in Fig.4 appears. A short-term analysis request (within one month), a mid-term analysis request (within six months), and a long-term analysis request (one year or more) which are set in phase (with time) are included in this course. Although the analysis of three phases like this is desirable in case of the gene analysis, it is possible to use the analysis of two phases. Gene information access center 6 clarifies the analytical content while explaining it for which the customer requests by using reference information shown in Fig.5 to Fig.8, and have the customer select.

The short-term analysis request course (within one month from the request) includes the followings as shown in Fig.5.

1)Genetic disease factor analysis
2)Cancer related gene analysis
3)Drug-metabolizing enzyme gene analysis
4)Chromosome analysis
5)Others

The mid-term analysis request course (within six months from the request) includes the followings as shown in Fig.6

1)Genetic disease gene (multi factor analysis).
2)Life-style related diseases factor gene analysis
3)Chronic disease related gene analysis
4)Drug-metabolizing enzyme gene analysis(wide range)
5)Chromosome analysis(details)
6)SNP (one base multi type) analysis
7)Others

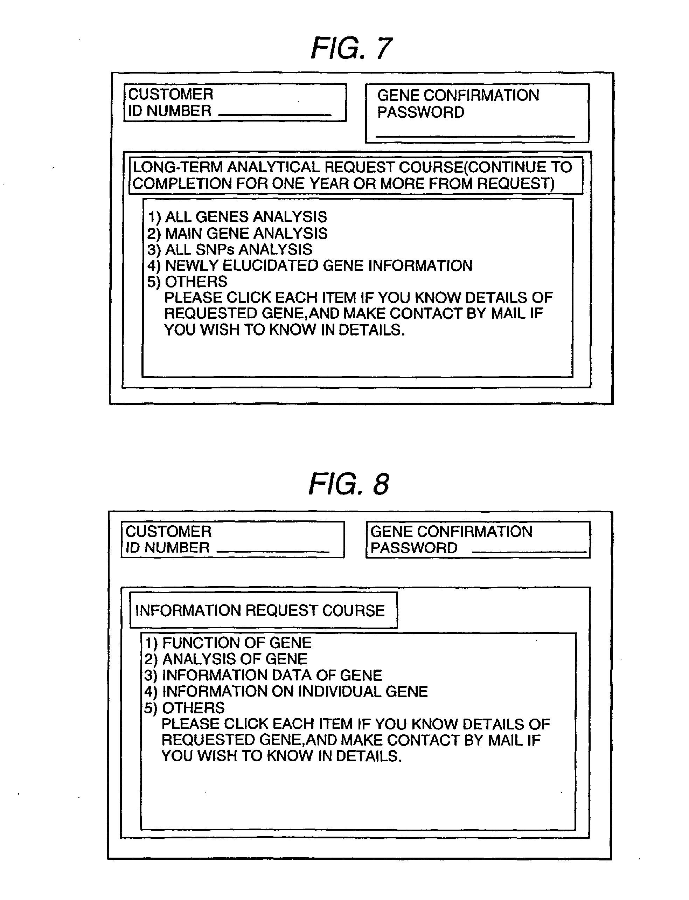

The long-term analysis request course (continues to the completion for one year or more from the request) includes the followings as shown in Fig.7.

1)All genes analysis
2)The main gene analysis
3)All SNPs analysis
4)Newly elucidated gene information
5)Others

The information request course includes the followings as shown in Fig.8.

1)Function of gene
2)Analysis of gene
3)Information data of gene
4)Information on individual gene
5)Others

Gene information access center 6 answers the doubt and the demand of the customer through the Internet etc. The rough estimate of the cost estimation is displayed on the customer access screen for the selected period based on the content of an analysis request and the report frequency (Fig.8).

After an analytical content is informed the customer, the approval screen is displayed. The account is decided by the followings.

1) selection period (short term, middle term, and long term), 2) number of gene to be analyzed, 3) difficulty of analysis gene, 3) situation of mutation degree, 4) information use period, 5) access center use frequency, 5) provision of an additional information (Mediation and introduction are included), etc. This is decided according to the add method

shown next.

$$T=[A(m1+m2+\cdots+mX)+B(n1+n2+\cdots+nX)+C(p1+p2+\cdots+pX)+D(t1+t2+\cdots+tX)+E]/L$$

Where, A, B, and C designate the accounting basic rate corresponding to the period course, mX, nX, and pX designates the basic charge of each analysis object gene in each course, D designates the basic rate for the information service, and tX designates the charge for individual information. Further, E designates other service charge that does not depend on the frequency. L designates the information service period, and T the total amount of the accounting per unit period. The accounting can be paid and the collection be made electronically through the network such as the Internet, etc. as shown in Table 2.

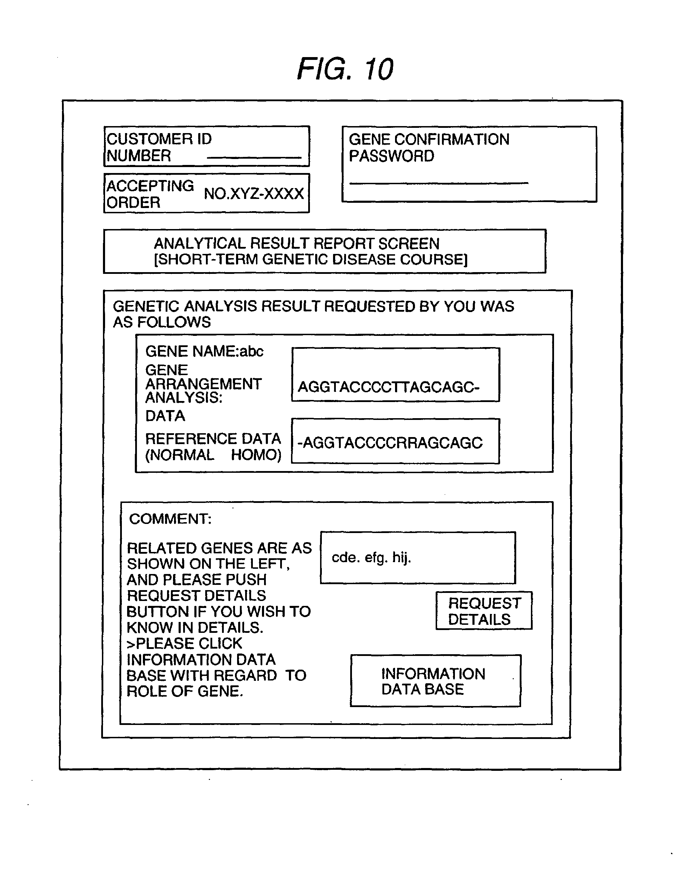

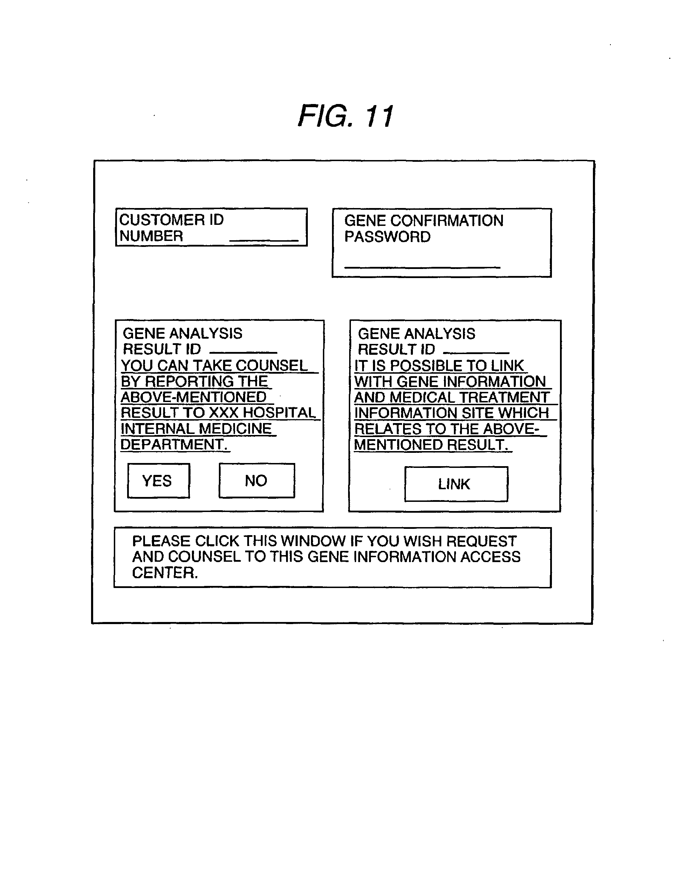

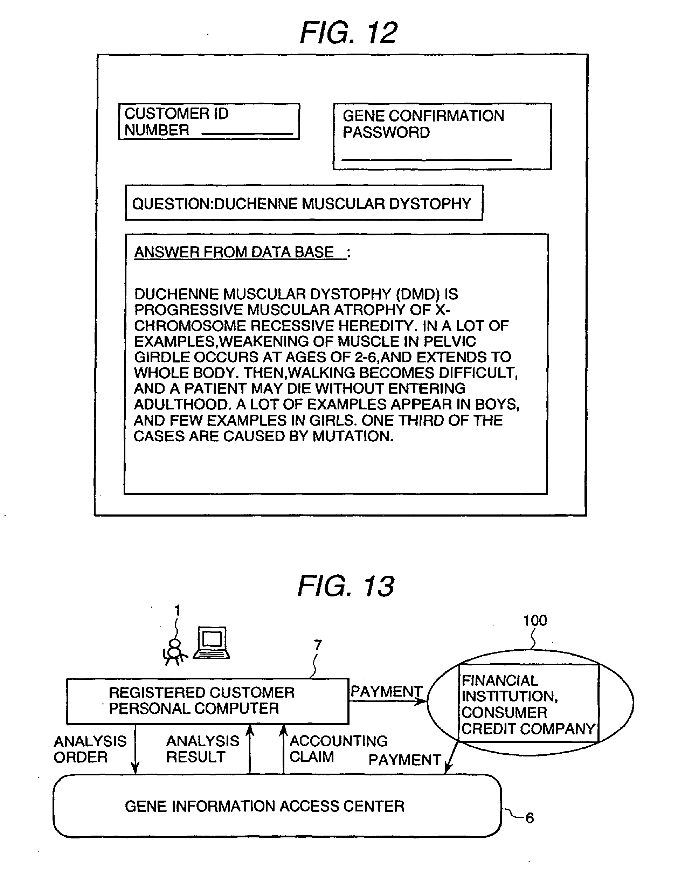

With regard to the information service, it is possible to add and/or change without being limited to the above-mentioned content. Reference information can be also provided to judge the value of these information (Fig.12). when the selection by the customer ends, the approval is obtained, and the order is done, the gene access center makes the plan to analyze the keeping gene and executes analytical work. In a long series of analysis, analytical work is started after the priority order is confirmed in advance by the customer. The obtained result is reported to the customer in the form shown in Fig.10. As a result, if the consultation of the medical treatment and the treatment information are occasionally requested from the customer side, a medical treatment organization is introduced and new information is provided to the medical treatment organization after agreement of the customer is obtained (Fig.11). Gene information access center 6 preserves the individual gene information obtained by the above-mentioned method for a long term. The name of the gene and the base sequence, etc. are accommodated in magnetic storage media such as the hard disk of the computer, MOs, and compact discs, and protected by the password for the individual confirmation. The information section in the gene information access center manages the information, the access to the customer, and the gene information in the access center. The service given to the customer is divided into many kinds of phases according to the period, the difficulty of the demand gene information and the use frequency, and the accounting is changed according to the phases. The accepting order system is set up so that the customer can select freely according to the desired information and the estimated amount of the accounting, and all information service method can be adopted.

Fig.13 shows a money payment method. In the figure, customer 1 ,that is, the gene donor requests the analysis from registration customer personal computer 7 to gene access center 6. The gene access center 6 demands the accounting with the notification of an analytical result. This accounting claim becomes one corresponding to three-phase analysis as mentioned above, and the unified price may be used when the umbrella contract is concluded. Customer 1 pay the accounting from the registered customer personal computer 7 to gene information access center 6 through financial institution and credit company 1.

According to the above-mentioned composition, the following method is provided.

A gene analysis information offering method in which analyzed gene sequence information is stored and provided, comprising: a step of collecting customer's gene, separating, and keeping; a step of selecting the content of analysis of gene sequence in phase by a customer; a step of receiving gene sequence analysis work, and storing data in a computer; and a step of transmitting gene sequence information, storing, and keeping.

Further, the following method is provided.

A gene analysis information offering method in which analyzed gene sequence information is stored and provided, comprising: a step of setting the content of gene sequence to at least three phases and setting charge corresponding to each of the phases; a step of selecting the content of analysis of gene sequence in phase by a customer; a step of accepting an order of gene sequence analysis based on the selection, and claiming accounting payment; a step of transmitting gene sequence information, storing, and keeping; and a step of attaching comments to the gene sequence information based on the demand of the customer, and providing the gene sequence information stored and kept or gene sequence information processed to an external organization along with comments.

The phased selection by the customer is performed to the kind and the number of gene, and gene related disease information.

The gene sequence is a base sequence of DNA.

Further, the following system is provided.

A gene analysis information offering system in which analyzed gene sequence information is stored and provided, comprising: a process computer having; a acceptance part which collects, separates, and keeps at least one gene, an input part which inputs phased selection about the content of analysis of gene sequence, an accepting order information input part which inputs accepting order information on the gene sequence analysis based on the selection, a processing part which claims the accounting payment corresponding to the selected phase, and a storage part which stores gene sequence information, and a network which connects the process computer, a gene donor's computer and an external organization computer; wherein the gene sequence information is transmitted to the gene donor computer,

EP 1 345 137 A1

the provi ding of the gene sequence information from the gene donor's computer to the external organization computer is made to be allowed.

The accounting payment claim and the payment work are done through said network.

Furthermore, the following system is provid ed.

A gene analysis information offering system in which analyzed gene sequence information is stored and provided, comprising: a process computer having; an input part which inputs phased selection about the content of analysis of gene sequence, an accepting order information input part which inputs accepting order information on the gene sequence analysis based on the selection, and a storage part which stores gene sequence information, wherein the computer issues a password made of gene base sequence signs corresponding to said phase.

The password is the combination of two or more gene base sequence signs.

Further, the following method is provided.

A gene analysis information offering method in which analyzed gene sequence information is stored and provided, comprising: a step of inputting phased selection about the content of analysis of the gene sequence, a step of inputting accepting order information on the gene sequence analysis based on the selection, a step of storing gene sequence information, and a step of issuing a password for identifying the attestation by combining two or more gene base sequence signs according to said selected phase.

Further, the following method is provided.

A gene attestation identifying method in which analyzed gene sequence information is stored and provided, comprising: a step of inputting phased selection about the content of analysis of the gene sequence, a step of storing gene sequence information, a step of issuing a password by combining two or more gene base sequ ence signs according to said selected phase, and a step of identifying the attestation of a gene donor by using the password.

## Claims

1. A gene analysis information offering method in which analyzed gene sequence information is stored and provided, comprising:

   a step of collecting customer's gene, separating, and keeping;
   a step of selecting the content of analysis of gene sequence in phase by a customer;
   a step of receiving gene sequence analysis work, and storing data in a computer; and
   a step of transmitting gene sequence information, storing, and keeping.

2. A gene analysis information offering method in which analyzed gene sequence information is stored and provided, comprising:

   a step of setting the content of gene sequence to at least three ph ases and setting charge corresponding to each of the phases;
   a step of selecting the content of analysis of gene sequence in phase by a customer;
   a step of accepting an order of gene sequence analysis based on the selection, and claiming accounting payment;
   a step of transmitting gene sequence information, storing, and keeping; and
   a step of offering comments to the gene sequence information based on the demand of the customer, and providing the gene sequence information stored and kept or gene sequence information processed to an external organization along with comments.

3. A gene analysis information offering method in which analyzed gene sequence information is stored and provided, wherein the phased selection by the customer is performed to the kind and the number of gene, and gene related disease information.

4. A gene analysis information offering method in which analyzed gene sequence information is stored and provided, wherein the gene sequence is a base sequence of DNA.

5. A gene analysis information offering system in which analyzed gene sequence information is stored and provided, comprising:

   a process computer having;

a acceptance part which collects, separates, and keeps at least one gene,
an input part which inputs phased selection about the content of analysis of gene sequence,
an accepting order information input part which inputs accepting order information on the gene sequence analysis based on the selection,
a processing part which claims the accounting payment corresponding to the selected phase, and
a storage part which stores gene sequence information, and

a network which connects the process computer, a gene donor 's computer and an external organization computer;

wherein the gene sequence information is transmitted to the gene donor computer, the providing of the gene sequence information from the gene donor's computer to the external organization computer is made to be allowed.

6. A gene analysis information offering system in which analyzed gene sequence information is stored and provided, wherein the accounting payment claim and the payment work are done through said network.

7. A gene analysis information offering system in which analyzed gene sequence information is stored and provided, comprising:

a process computer having;

an input part which inputs phased selection about the content of analysis of gene sequence,
an accepting order information input part which inputs accepting order information on the gene sequence analysis based on the selection, and
a storage part which stores gene sequence information,

wherein the computer issues a password made of gene base sequence signs corresponding to said phase.

8. A gene analysis information offering system in which analyzed gene sequence information is stored and provided, wherein said password is the combination of two or more gene base sequence signs.

9. A gene analysis information offering method in which analyzed gene sequence information is stored and provided, comprising:

a step of inputting phased selection about the content of analysis of the gene sequence,
a step of inputting accepting order information on the gene sequence analysis based on the selection,
a step of storing gene sequence information, and
a step of issuing a password for identifying the attestatio n by combining two or more gene base sequence signs according to said selected phase.

10. A gene attestation identifying method in which analyzed gene sequence information is stored and provided, comprising:

a step of inputting phased selection about the content of analysis of the gene sequence,
a step of storing gene sequence information,
a step of issuing a password by combining two or more gene base sequence signs according to said selected phase, and
a step of identifying the attestation of a gene do nor by using the password.

# FIG. 1

CUSTOMER ~1

EXTERNAL ORGANIZATION (EXTERNAL MEDICAL TREATMENT ORGANIZATION) ~2

APPLICATION OF VISIT

INTERMEDIATION OF INFORMATION

ACCEPTANCE SECTION
1) BLOOD DRAWING
2) WHITE BLOOD CORPUSCLE SEPARATION
3) GENE SEPARATION
4) SUBDIVISION PRESERVATION
5) ANALYSIS FOR INDIVIDUAL IDENTIFICATION ~3

PRESERVATION AND ACCEPTING ORDER ANALYSES

ANALYSIS AND INFORMATION SECTION
1) GENE AND RESULT PRESERVATION
2) SHORT-TERM ANALYSIS
3) MID-TERM ANALYSIS
4) LONG-TERM ANALYSIS
5) INFORMATION SERVICE ~4

CUSTOMER REGISTRATION SECTION (GENE PASSWORD ISSUE FOR MEMBER) ~5

ORDER,INFORMATION SERVICE,AND COST CLAIM ~9

VIA INTERNET     VIA INTERNET

CON-SULTATION OF MEDICAL TREATMENT

PASSWORD ISSUE ~8

REGISTRATION CUSTOMER PERSONAL COMPUTER ~7

GENE INFORMATION ACCESS CENTER ~6

# FIG. 2

REGISTRATION SCREEN — 11

↓

SELECTION MENU SCREEN — 12

↓

SELECTION COURSE SCREEN — 13

↓

ANALYTICAL REQUEST AND PROCEDURE SCREEN — 14 ⟷ GENE EXPLANATION SCREEN — 15

↓

METHOD OF PAYING COST AND PROCEDURE SCREEN — 16 ⟷ PAYMENT TERMS AND RESULT REPORT CONDITION EXPLANATION — 17

↓

RESULT REPORT SCREEN — 18

⟷ RESULT INTERPRETATION EXPLANATION SCREEN — 19

⟷ CONSULTATION REQUEST SCREEN — 20

⟷ MEDICAL TREATMENT ORGANIZATION INTRODUCTION SCREEN — 21

⟷ LINK INFORMATION DATA BASE SCREEN — 22

## *FIG. 3*

GENETIC ANALYSIS SERVICE
REQUEST SCREEN

CUSTOMER
ID NUMBER _____

GENE CONFIRMATION
PASSWORD

_____

PLEASE INPUT YOUR IDENTIFICATION NUMBER AND
GENE CONFIRMATION PASSWORD,AND PLEASE CLICK
FOLLOWING TRANSMISSION BUTTON AFTER
CONFIRMING NUMBER AND PASSWORD.

TRANSMISSION

## *FIG. 4*

GENETIC ANALYSIS SERVICE SELECTION SCREEN

PLEASE SELECT SERVICE YOU WANT

1) SHORT-TERM ANALYTICAL REQUEST(WITHIN ONE
MONTH)
2) MID-TERM ANALYTICAL REQUEST(WITHIN SIX MONTHS)
3) LONG-TERM ANALYTICAL REQUEST(ONE YEAR OR
MORE)
4) LONG-TERM GENE PRESERVATION
5) GENE INFORMATION SERVICE
6) MEDICAL TREATMENT ORGANIZATION INTRODUCTION

## FIG. 5

CUSTOMER
ID NUMBER _____

GENE CONFIRMATION
PASSWORD
_____

SHORT-TERM ANALYTICAL REQUEST COURSE
(WITHIN ONE MONTH FROM REQUEST)

1) GENETIC DISEASE FACTOR ANALYSIS
2) CANCER RELATED GENE ANALYSIS
3) DRUG-METABOLIZING ENZYME GENE ANALYSIS
4) CHROMOSOME ANALYSIS
5) OTHERS
   PLEASE CLICK EACH ITEM IF YOU KNOW DETAILS OF
   REQUESTED GENE,AND MAKE CONTACT BY MAIL IF
   YOU WISH TO KNOW IN DETAILES.

## FIG. 6

CUSTOMER
ID NUMBER _____

GENE CONFIRMATION
PASSWORD
_____

MID-TERM ANALYTICAL REQUEST COURSE
(WITHIN SIX MONTHS FROM REQUEST)

1) GENETIC DISEASE GENE(MULTI FACTOR ANALYSIS)
2) LIFE-STYLE RELATED DESEASES FACTOR GENE
   ANALYSIS
3) CHRONIC DISEASE RELATED GENE ANALYSIS
4) DRUG-METABOLIZING ENZYME GENETIC ANALYSIS
   (WIDE RANGE)
5) CHROMOSOME ANALYSIS(DETAILS)
6) SNP(ONE BASE MULTI TYPE) ANALYSIS
7) OTHERS
   PLEASE CLICK EACH ITEM IF YOU KNOW DETAILS OF
   REQUESTED GENE,AND MAKE CONTACT BY MAIL IF
   YOU WISH TO KNOW IN DETAILES.

## FIG. 7

CUSTOMER
ID NUMBER _____

GENE CONFIRMATION
PASSWORD
_____

LONG-TERM ANALYTICAL REQUEST COURSE(CONTINUE TO
COMPLETION FOR ONE YEAR OR MORE FROM REQUEST)

1) ALL GENES ANALYSIS
2) MAIN GENE ANALYSIS
3) ALL SNPs ANALYSIS
4) NEWLY ELUCIDATED GENE INFORMATION
5) OTHERS
   PLEASE CLICK EACH ITEM IF YOU KNOW DETAILS OF
   REQUESTED GENE,AND MAKE CONTACT BY MAIL IF
   YOU WISH TO KNOW IN DETAILS.

## FIG. 8

CUSTOMER
ID NUMBER _____

GENE CONFIRMATION
PASSWORD _____

INFORMATION REQUEST COURSE

1) FUNCTION OF GENE
2) ANALYSIS OF GENE
3) INFORMATION DATA OF GENE
4) INFORMATION ON INDIVIDUAL GENE
5) OTHERS
   PLEASE CLICK EACH ITEM IF YOU KNOW DETAILS OF
   REQUESTED GENE,AND MAKE CONTACT BY MAIL IF
   YOU WISH TO KNOW IN DETAILS.

# FIG. 9

CUSTOMER ID
NUMBER _____

GENE CONFIRMATION
PASSWORD

_____

ANALYSIS REQUEST ORDER SHEET

ORDER CONTENTS

NAME OF
COURSE

REQUESTED
GENE NAME

REQUESTED
DESEASE NAME

ESTIMATION
OF COSTS

TOTAL ___

HERE IS
SPECIFICATION

DEMAND FOR ANALYSIS
RESULT REPORT

○ DETAILED ANALYSIS
○ BRIEF ANALYSIS RESULT

I AGREE TO CONTENTS OF ANALYSIS REQUEST AND
PAYMENT OF COSTS, AND ORDER ALANLYSIS

YES    NO

# FIG. 10

CUSTOMER ID NUMBER _____

GENE CONFIRMATION PASSWORD
_____

ACCEPTING ORDER NO.XYZ-XXXX

ANALYTICAL RESULT REPORT SCREEN
[SHORT-TERM GENETIC DISEASE COURSE]

GENETIC ANALYSIS RESULT REQUESTED BY YOU WAS AS FOLLOWS

GENE NAME:abc

GENE ARRANGEMENT ANALYSIS: DATA

AGGTACCCCTTAGCAGC-

REFERENCE DATA (NORMAL HOMO)

-AGGTACCCCRRAGCAGC

COMMENT:

RELATED GENES ARE AS SHOWN ON THE LEFT, AND PLEASE PUSH REQUEST DETAILS BUTTON IF YOU WISH TO KNOW IN DETAILS.
>PLEASE CLICK INFORMATION DATA BASE WITH REGARD TO ROLE OF GENE.

cde. efg. hij.

REQUEST DETAILS

INFORMATION DATA BASE

# FIG. 11

| CUSTOMER ID NUMBER _____ | GENE CONFIRMATION PASSWORD _____ |
|---|---|

GENE ANALYSIS
RESULT ID _____
YOU CAN TAKE COUNSEL
BY REPORTING THE
ABOVE-MENTIONED
RESULT TO XXX HOSPITAL
INTERNAL MEDICINE
DEPARTMENT.

| YES | NO |
|---|---|

GENE ANALYSIS
RESULT ID _____
IT IS POSSIBLE TO LINK
WITH GENE INFORMATION
AND MEDICAL TREATMENT
INFORMATION SITE WHICH
RELATES TO THE ABOVE-
MENTIONED RESULT.

| LINK |
|---|

PLEASE CLICK THIS WINDOW IF YOU WISH REQUEST
AND COUNSEL TO THIS GENE INFORMATION ACCESS
CENTER.

## FIG. 12

CUSTOMER ID
NUMBER _____

GENE CONFIRMATION
PASSWORD

_____

QUESTION:DUCHENNE MUSCULAR DYSTOPHY

ANSWER FROM DATA BASE : 

DUCHENNE MUSCULAR DYSTOPHY (DMD) IS
PROGRESSIVE MUSCULAR ATROPHY OF X-
CHROMOSOME RECESSIVE HEREDITY. IN A LOT OF
EXAMPLES,WEAKENING OF MUSCLE IN PELVIC
GIRDLE OCCURS AT AGES OF 2-6,AND EXTENDS TO
WHOLE BODY. THEN,WALKING BECOMES DIFFICULT,
AND A PATIENT MAY DIE WITHOUT ENTERING
ADULTHOOD. A LOT OF EXAMPLES APPEAR IN BOYS,
AND FEW EXAMPLES IN GIRLS. ONE THIRD OF THE
CASES ARE CAUSED BY MUTATION.

## FIG. 13

1

100

REGISTERED CUSTOMER
PERSONAL COMPUTER                    7

PAYMENT

FINANCIAL
INSTITUTION,
CONSUMER
CREDIT COMPANY

ANALYSIS
ORDER

ANALYSIS
RESULT

ACCOUNTING
CLAIM

PAYMENT

GENE INFORMATION ACCESS CENTER                    6

<div align="center">

**INTERNATIONAL SEARCH REPORT**

</div>

| | International application No. |
|---|---|
| | PCT/JP00/08489 |

**A. CLASSIFICATION OF SUBJECT MATTER**
Int.Cl$^7$   G06F17/60

According to International Patent Classification (IPC) or to both national classification and IPC

**B. FIELDS SEARCHED**

Minimum documentation searched (classification system followed by classification symbols)
Int.Cl$^7$   G06F17/60, 17/30, 19/00      Int.Cl$^7$   A61B 5/00

Documentation searched other than minimum documentation to the extent that such documents are included in the fields searched
Jitsuyo Shinan Koho          1926-1996    Jitsuyo Shinan Toroku Koho  1996-2001
Kokai Jitsuyo Shinan Koho  1971-2001    Toroku Jitsuyo Shinan Koho  1994-2001

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, 9-311902, A (Setsuo OGISO),<br>02 December, 1997 (02.12.97),<br>Full text; Figs. 1 to 5   (Family: none) | 1-3,5-10 |
| Y<br>X | JP, 10-272123, A (Hitachi, Ltd.),<br>13 October, 1998 (13.10.98),<br>Full text; Figs. 1 to 5   (Family: none) | 1-3,5-10<br>4 |
| Y | JP, 11-353404, A (Hitachi, Ltd.),<br>24 December, 1999 (24.12.99),<br>Full text; Figs. 1 to 18   (Family: none) | 3 |
| Y | JP, 10-151125, A (Corp. Miyuki K.K.),<br>24 December, 1999 (24.12.99),<br>Full text; Figs. 1 to 5   (Family: none) | 7-10 |
| A | JP, 2000-262479, A (Hitachi, Ltd.),<br>26 September, 2000 (26.09.00),<br>Full text; Figs. 1 to 10   (Family: none) | 1-10 |

☒ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 February, 2001 (15.02.01) | 27 February, 2001 (27.02.01) |

| Name and mailing address of the ISA/<br>Japanese Patent Office | Authorized officer |
|---|---|
| Facsimile No. | Telephone No. |

Form PCT/ISA/210 (second sheet) (July 1992)

## INTERNATIONAL SEARCH REPORT

| International application No. |
| --- |
| PCT/JP00/08489 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP, 11-85875, A (Dainippon Printing Co., Ltd.), 30 March, 1999 (30.03.99), Full text; Figs. 1 to 6   (Family: none) | 1-10 |

Form PCT/ISA/210 (continuation of second sheet) (July 1992)